(19) Europäisches Patentamt / European Patent Office / Office européen des brevets

(11) **EP 4 449 976 A1**

(12) **EUROPEAN PATENT APPLICATION**

(43) Date of publication:
**23.10.2024 Bulletin 2024/43**

(21) Application number: **23169009.0**

(22) Date of filing: **20.04.2023**

(51) International Patent Classification (IPC):
**A61B 5/00** (2006.01)

(52) Cooperative Patent Classification (CPC):
**A61B 5/4094; A61N 1/0476; A61N 1/36025;
A61N 1/36064; G16H 20/30; G16H 50/50;**
A61B 5/383; A61B 5/7278

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Université d'Aix Marseille
13007 Marseille (FR)**
• **Institut National de la Santé et de la Recherche
Médicale
75013 Paris (FR)**

(72) Inventors:
• **JIRSA, Viktor
13400 Marseille (FR)**

• **TRIEBKORN, Paul
13008 Marseille (FR)**
• **WANG, Huifang
13400 Aubagne (FR)**
• **DUMA, Gian-Marco
13010 Marseille (FR)**
• **DOLLOMAJA, Borana
13005 Marseille (FR)**
• **WILLIAMSON, Adam
13010 Marseille (FR)**

(74) Representative: **Macquet, Christophe
Macquet & Associés
Arche des Dolines
7, rue Soutrane
06560 Sophia Antipolis (FR)**

(54) **A METHOD AND SYSTEM FOR ESTIMATING AN EPILEPTOGENIC ZONE NETWORK**

(57)     The invention relates to a method and a system for estimating an epileptogenic zone network in a brain of an epileptic patient. According to the invention, the method comprises the steps of providing a mathematical model of an epileptogenic zone, wherein the mathematical model is provided with epileptogenicity parameters; loading the mathematical model in the virtual brain to create a virtual epileptic brain; acquiring three-dimensional structural data representative of the anatomy of the brain of the epileptic patient; personalising the virtual epileptic brain according to the structural data to obtain a personalised brain model; acquiring functional data recordings representative of epileptic seizures in the brain of the epileptic patient, the functional data recordings being acquired non-invasively by performing a deep brain temporal interference stimulation of the brain of the patient; and inverting the personalised brain model of the patient to infer the epileptogenicity parameters specific to the patient for the various zones or nodes of the virtual epileptic model of the brain of the patient.

FIG. 2A

**Description**

FIELD OF THE INVENTION

The invention relates to a method and a system for estimating an epileptogenic zone network in a brain of an epileptic patient. According to the invention, the method comprises the steps of providing a virtual brain, the virtual brain being a computerized platform modelling various zones or nodes of a primate brain, preferably human brain, and connectivity between said zones or nodes; providing a mathematical model of an epileptogenic zone and of a propagation of an epileptic discharge from an epileptic zone to a propagation zone, the model describing the onset, the time-course and the offset of an epileptic discharge, wherein the mathematical model is provided with epileptogenicity parameters; loading the mathematical model in the virtual brain to create a virtual epileptic brain; acquiring three-dimensional structural data representative of the anatomy of the brain of the epileptic patient; and personalising the virtual epileptic brain according to the structural data to obtain a personalised brain model.

BACKGROUND OF THE INVENTION

For drug-resistant focal epilepsy, in order to estimate the location of the epileptogenic zone (EZ) network in the brain of a patient, the usual clinical routine generally includes the implementation of electroencephalographic (EEG) and/or stereo-electroencephalographic (SEEG) examinations.

EEG examinations require the use of an helmet, that encloses electrodes, the electrodes being in close physical contact with the surface of the scalp, this close contact being achieved using a gel that is drying along time. The duration of EEG examinations is thus limited in time. Practically, their duration is of about 1 hour. In such limited time, the probability to effectively observe an epileptic seizure for a patient is quite low.

This is the reason why stimulation-induced seizures remain an integral part for EZ diagnosis and have proven to be a significant predictive factor for good outcome. Among 142 patients with high frequency (50Hz) induced seizure, 62.7% has no seizure recurrence after surgery. Among 120 patients with low frequency (1Hz) induced seizure, 71.7% has no seizure recurrence. But among 84 patients with no induced seizure, 53.6% has no seizure recurrence.

SEEG examinations are thus preferred because they allow triggering epileptic seizures in the patient brain that, in turn, allow to estimate the location of the EZ network. However, SEEG examinations necessitate the implantation of electrodes in the patient brain, which is invasive. The most common complications of SEEG examinations are hemorrhagic, with a pooled prevalence of 1%, or infectious, with a pooled prevalence of 0.8%.

Hence, practically, EEG examinations are, at first, preferred and carried out. Even if epileptic seizures are not observed, EEG examinations may allow to obtain at least certain signals, which permit to determine if the EZs may be at least identified or not with a certain probability. The current EEG examinations help the clinicians form the first EZ network hypothesis and access possibility for the implantation of SEEG examinations if the surgery is required.

Scientific and clinical societies are working on possible non-invasive diagnosis and treatment options for epilepsy patients with similar or even better outcome as the SEEG invasive ones.

Also, advanced anatomic neuroimaging techniques, such as Magnetic Resonance Imaging (MRI) and Diffusion Tensor Imaging (DTI), and functional recordings, such as EEG, SEEG and magnetoencephalography (MEG), is allowing to build a high-resolution whole brain model for each individual patient. It has the ability to do personalised diagnosis, such as the estimation of epileptogenic zones, and prediction and control of personalised treatment, such as the guidance of stimulation and intervention.

SUMMARY OF THE INVENTION

Accordingly, a need exists for estimate the location of EZ networks, non-invasively, i.e. without implantation of intracranial electrodes.

In accordance with a first aspect, the invention concerns a method for estimating an epileptogenic zone network in a brain of an epileptic patient, comprising

providing a virtual brain, the virtual brain being a computerized platform modelling various zones or nodes of a primate brain, preferably human brain, and connectivity between said zones or nodes;
providing a mathematical model of an epileptogenic zone and of a propagation of an epileptic discharge from an epileptogenic zone to a propagation zone, the model describing the onset, the time-course and the offset of an epileptic discharge,
wherein the mathematical model is provided with epileptogenicity parameters;
loading the mathematical model in the virtual brain to create a virtual epileptic brain;
acquiring three-dimensional structural data representative of the anatomy of the brain of the epileptic patient;

personalising the virtual epileptic brain according to the structural data to obtain a personalised virtual epileptic brain model of the patient;

acquiring functional data recordings representative of epileptic seizures in the brain of the epileptic patient, the functional data recordings being acquired non-invasively by performing a deep brain temporal interference stimulation of the brain of the patient;

inverting the personalised virtual epileptic brain model of the patient to infer the epileptogenicity parameters specific to the patient for the various zones or nodes of the personalised virtual epileptic brain model of the patient;

simulating at least one epileptogenic seizure in the personalised virtual epileptic brain model wherein the epileptogenic parameters are inferred; and

estimating the epileptogenic zone network in the brain of the epileptic patient.

Preferentially, - the personalised virtual epileptic brain is a high-resolution model that simulates a neural source activity with a spatial resolution of less than 5 mm$^2$; - the spatial resolution is of approximately 1 mm$^2$; - for performing the deep brain temporal interference stimulation of the brain of the patient, it is provided at least two pairs of stimulation electrodes, each pair of electrodes providing an electric signal at a carrier frequency generating an envelope frequency for stimulation at a point in space in the brain of the patient, the envelope frequency being equal to the difference $\Delta f$ between the two electric signal frequencies provided; - for performing the deep brain temporal interference stimulation of the brain of the patient, it is provided at least four pairs of stimulation electrodes, each pair of electrodes providing an electric signal at a carrier frequency, wherein the mean value between the carrier frequency of a first pair of electrodes and the carrier frequency of a second pair of electrodes defines a first mean carrier frequency, and the mean value between the carrier frequency of a third pair of electrodes and the carrier frequency of a fourth pair of electrodes defines a second mean carrier frequency, and wherein the difference between said first and second mean carrier frequencies is at least 200 Hz, preferably at least 500 Hz even more preferably at least 800 Hz; - each electric signal provided by each pair of electrodes has a carrier frequency of at least 800 Hz, preferably at least 900 Hz, more preferably at least 1000 Hz; - the electrodes are applied at different locations on a surface of a scalp of the patient skull; - the first pair of electrodes and the second pair or electrodes generate a first envelope frequency, said first envelope frequency being equal to the difference between the electric signal frequency provided by the first pair of electrodes and the electric signal frequency provided by the second pair of electrodes; - the third pair of electrodes and the fourth pair of electrodes generate a second envelope frequency, said second envelope frequency being equal to the difference between the electric signal frequency provided by the third pair of electrodes and the electric signal frequency provided by the fourth pair of electrodes; and wherein, the first envelope frequency is equal to the second envelope frequency; - the structural data comprise geometry data of the cortex of the patient's brain and structural connectivity within the cortex of the patient's brain; - the geometry data is extracted from T1-weighted MRI and the structural connectivity is extracted from tractography on diffusion weighted MRI data; - the method further comprises providing functional data recordings acquired invasively, which are representative of epileptic seizures in the brain of the epileptic patient; providing an integration module; combining the functional data recordings acquired non-invasively by performing a deep brain temporal interference stimulation and the functional data recordings acquired invasively in the integration module; - the functional data recordings comprise electro-encephalographic data recordings acquired by performing temporal interference and electro-encephalographic data recordings acquired by stereo-electro-encephalography; - the mathematical model of an epileptogenic zone and of a propagation of an epileptic discharge from an epileptic zone to a propagation zone comprises a plurality of state variables and a parameter I$_{\text{stim}}$ representative of the stimulation time course; - the mathematical model is provided by the set of equations as follows:

$$\dot{x}_1 \;=\; y_1 - f_1(x_1) - z + I_{ext1} + I_{stim}$$

$$\dot{y}_1 \;=\; c - dx_1^2 - y_1$$

$$\dot{z} \;=\; r(4(x_1 - x_0 + \text{nH}(\text{m} - m_{thresh})) - z) + f_3(z) + K\sum_{j=1}^{N} C_{i,j}\,(x_1^j - x_1^i)$$

$$\dot{m} \;=\; r(k|I_{stim}| - m)$$

where

$$f_1(x_1) \quad = \quad \begin{cases} ax_1^3 - bx_1^2 & \text{if } x_1 < 0 \\ -(m + 0.6(z-4)^2)x_1 & \text{if } x_1 \geq 0 \end{cases}$$

$$f_3(z) \quad = \quad \begin{cases} -0.1z^7 & \text{if } z < 0 \\ 0 & \text{if } z \geq 0 \end{cases}$$

wherein the state variables $x_1$ and $y_1$ describe the activity of neural populations on a fast time scale and can model fast discharges, the oscillation of the slow permittivity variable z drives the system autonomously between ictal and interictal states, variable m describes the stimulation effect, the parameter $x_0$ indicates the degree of excitability and directly controls the dynamics of the neural population to produce the seizure or not, The default parameters are $I_{ext1}$ = 3.1, c = 1, d = 5, r = 0.00035, a = 1, b = 3, the coupling between nodes of the network is defined by $C_{i,j}$, which comes from the structural connectivity, K scales is the connectivity and, for stimulation effects, $I_{stim}$ describes stimulation input; and
- the brain model inversion implements Hamiltonian Monte Carlo sampling techniques from Bayesian inference methods.

In accordance with a second aspect, the invention concerns a system for estimating an epileptogenic zone network in a brain of an epileptic patient, comprising

a virtual brain, the virtual brain being a computerized platform modelling various zones or nodes of a primate brain and connectivity between said zones or nodes;
a mathematical model of an epileptogenic zone and of a propagation of an epileptic discharge from an epileptic zone to a propagation zone, the model describing the onset, the time-course and the offset of an epileptic discharge, the mathematical model being provided with epileptogenicity parameters;
the mathematical model being loaded in the virtual brain to create a virtual epileptic brain;
acquired three-dimensional structural data representative of the anatomy of the brain of the epileptic patient;
the virtual epileptic brain being personalised according to the structural data to obtain a personalised virtual epileptic brain model of the patient;
a deep brain temporal interference stimulation device;
acquired functional data recordings representative of epileptic seizures in the brain of the epileptic patient, the functional data recordings being acquired non-invasively by performing a deep brain temporal interference stimulation of the brain of the patient using the deep brain temporal interference stimulation device;
a module for inverting the personalised virtual epileptic brain model to infer the epileptogenicity parameters specific to the patient for the various zones or nodes of the personalised virtual epileptic brain model of the patient;
means for simulating at least one epileptogenic seizure in the personalised virtual epileptic brain model where the epileptogenic parameters are inferred; and
means for estimating the epileptogenic zone network in the brain of the epileptic patient.

BRIEF DESCRIPTION OF THE DRAWINGS

Other features and aspects of the present invention will be apparent from the following description and the accompanying drawings, in which:

Fig. 1A illustrates the principle of a deep brain temporal interference stimulation for the implementation of the method according to the invention;
Figs. 1B and 1C illustrate an example of a mode for carrying out deep brain temporal interference stimulation for the implementation of the method, in which 4 pairs of electrodes are used, defining a quadrupole, those pairs of electrodes being noted E1A/E1B, E2A/E2B, E3A/E3B and E4A/E4B in Fig. 1B;
Fig. 1D compares the modulation the index profiles of dipole and octupole configurations for deep brain temporal interference stimulation, for the implementation of the invention;
Figs. 2A and 2B illustrate a workflow of the method according to the invention;
Figs. 3A to 3G illustrate a method of estimating an epileptogenic zone, where the functional data are acquired from SEEG stimulation;
Figs. 4A to 4F illustrate the same, but according to the invention, wherein the functional data are acquired non-invasively by performing a deep brain temporal interference stimulation of the brain of the patient according to the invention;
Figs. 5A to 5D illustrate the implementation of an integration module, for the implementation of a particular mode for carrying out the method according to the invention; and
Figs. 6A and 6B the time-series of the Epileptor™ stimulation equations by inducing epileptic seizures through SEEG stimulation (Fig. 6A) or Temporal Interference (TI).

DETAILLED DESCRIPTION OF THE INVENTION

This invention describes a high-resolution brain model for estimating, i.e. for identifying the location, of an epileptogenic zone (EZ) network in a brain of an epileptic patient. For comparison purpose, the application of invasive SEEG stimulation is investigated, as well as an innovative non-invasive procedure called Temporal Interference (TI).

The estimation of the EZ network according to the invention is non-therapeutic but is an important diagnosis for drug-resistant focal epilepsy and plays a pivotal role for treatment and intervention. Whole brain modelling provides a natural way for personalised diagnosis by integrating patient-specific brain geometry and structural connectivity from anatomical neuroimaging such as MRI, and dynamic activity from functional recordings such as EEG or SEEG. According to the invention, seizure-like events demonstrate rich spatial and temporal features from functional recordings, which are exploited to estimate the EZ network. Stimulation induced seizures become complementary information when not enough spontaneous seizures are recorded. In this description, invasive SEEG stimulation is considered as the current most practical approach, and deep brain TI stimulation as the approach according to the invention, for non-invasive diagnosis and treatment. According to the invention, a framework is provided to estimate the EZ network from stimulation induced seizures based on whole brain network modelling. Model inversion may use Hamiltonian Monte Carlo sampling to estimate the EZ network. The high-resolution neural field model is based on patient-specific neuroimaging and functional recordings, which provide the ground truth for testing the framework.

The method according to the invention is based on a personalised high resolution modelling on stimulation for a particular patient. It is called the Virtual Epilepsy Patient (VEP) for stimulation. A personalised high resolution modelling of this patient is built, who underwent both SEEG and TI stimulations, and the high resolution source signals are projected to the EEG and SEEG data. Then, the EZ network may be estimated from simulated SEEG and EEG data, and the estimation can be validated in terms of the ground truth of the EZ network from high resolution simulation on the source level. An integration module is also introduced to refine the estimation of the EZ network by combining SEEG/EEG signals.

Deep Brain Temporal Interference Stimulation

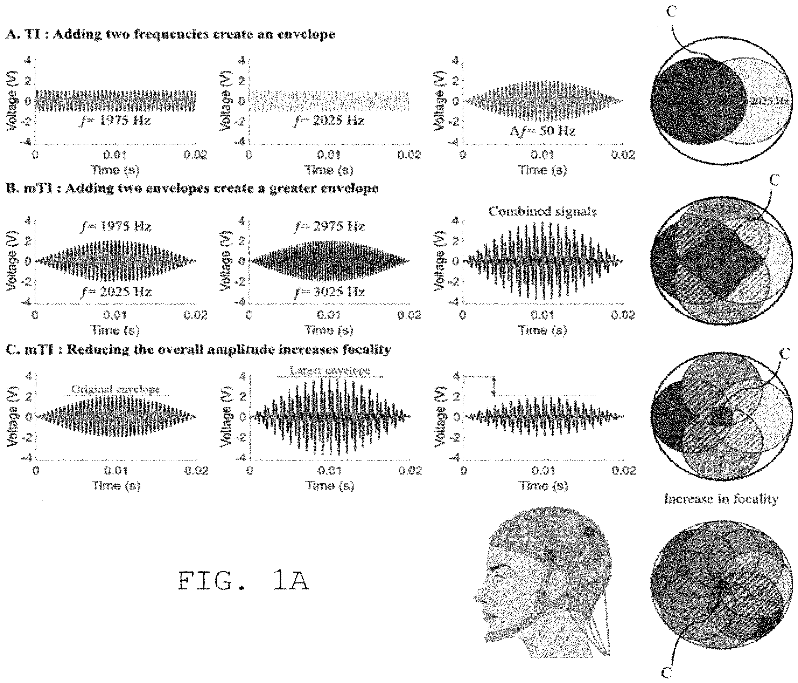

The TI stimulation technique is non-invasive. It relies on the ability of high frequency currents to penetrate living tissue relatively unhindered compared to low frequency currents, inferior to 800 Hz, as tissue permittivity tend to increase with frequency. The TI principle is based on the interaction of two high frequency electric currents - or signals - each of them being provided at a carrier frequency of at least 900 Hz, preferably of at least 1000 Hz, but suffering from a slight difference. This frequency difference creates a temporal interference in a zone C that is both predictable and regular, at a frequency equal to the difference between two carrier frequencies, as seen in Fig. 1A, panel A wherein the carrier frequencies are of 1975 Hz and 2025 Hz and the frequency difference is of 50 Hz. The neuronal or muscular tissues will only be modulated when they are simultaneously stimulated by these two frequencies, namely when the tissue experiences the interference, as it is generally accepted that frequencies above 900 Hz should not stimulate tissue.

It is to be noted that all the tissue, which experiences the interference, can be modulated when the amplitude of the envelope exceeds a threshold. However, using only two pairs of electrodes offers little freedom in the spatial configuration of the stimulators, which often results in a large area of effective stimulation, especially in deep structures. In order to avoid to modulate unwanted nodes in the brains or neighboring nerves in the case of peripheral nerve stimulation, the number of stimulating pairs is preferentially increased to improve the focality of stimulation, namely the ability to modulate a targeted area without disturbing the surrounding area. This relies on the addition of several envelopes having the maximum amplitude of envelope at the same spot, as shown in Fig. 1A, Panel B.

With supplementary pairs of stimulation electrodes, the current provided by each pair of electrodes can be turned down to achieve the same effect at the targeted zone C, while offering a better focality, as shown in Fig. 1A, Panel C. When performing this multipolar interference technique, noted mTI in Fig. 1A, a few things should however be considered. The frequency sets chosen to create envelopes should respect certain criteria: each envelope should be of the same frequency, the difference between the means of the carrier frequencies of associated pairs used to create the envelopes should be at least 200 Hz, in order to prevent the creation of unwanted interferences. The phase of each envelope should be considered at the targeted zone. It should be synchronous at this targeted zone to deliver the most efficient stimulation, but asynchronous outside the targeted zone, which will decrease the efficiency of stimulation, thus also improving the focality of stimulation. This technique works better when the stimulating pairs are in different planes, allowing to really "lock" the targeted zone in 3 dimensions.

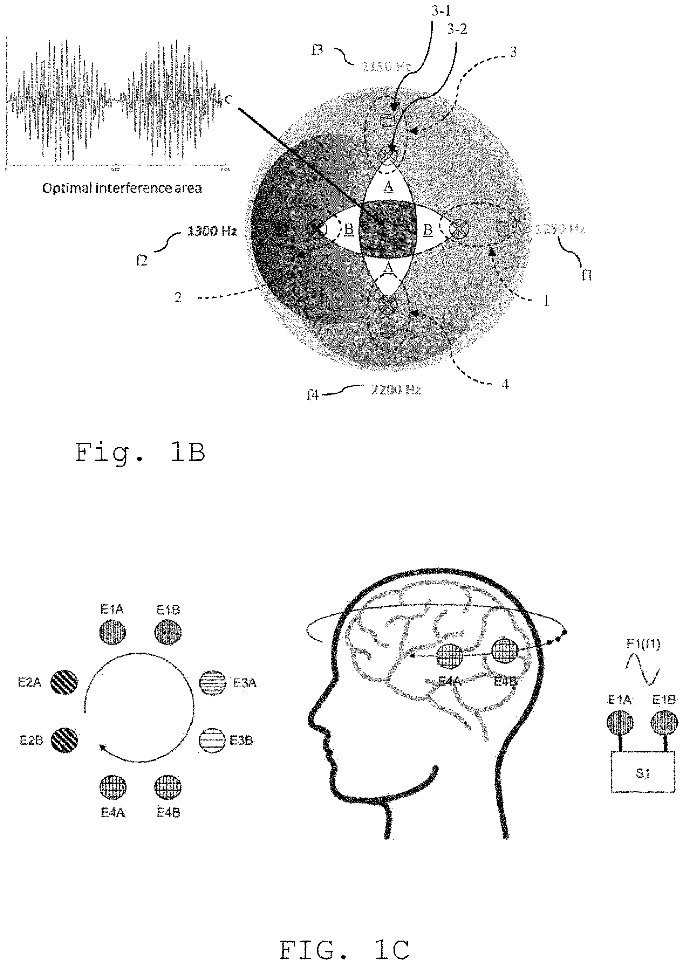

Fig. 1B illustrates an embodiment of a deep brain stimulation system for acquiring functional data recordings representative of epileptic seizures in the brain of the epileptic patient implementing mTI. As illustrated in Fig. 1B, the system comprises 4 stimulation pairs of electrodes 1, 2, 3, 4, a first stimulation pair of electrodes 1, a second stimulation pair of electrodes 2, a third stimulation pair of electrodes 3, and a fourth stimulation pair of electrodes 4, defining a quadrupole. The pairs of electrodes are positioned on different sides of the brain scalp, each stimulation pair comprising one electrode 3-1 and its associated return electrode 3-2. It is to be noted that, in Fig. 1B, the electrodes of a pair of electrodes are schematized by a cylinder and a circle containing a cross. For example, the stimulation pair of electrodes referenced 3 in Fig. 1B comprises two electrodes that are referenced 3-1 and 3-2. The electrode 3-2, that is schematized by a circle containing a cross, is the return electrode.

Each pair of electrodes provides an electric signal at a carrier frequency. The carrier frequency of the first stimulating pair of electrodes 1 is of 1250 Hz. The carrier frequency of the second stimulation pair 2 of electrodes is 1300 Hz. The carrier frequency of the third stimulation pair of electrodes 3 is 2150 Hz. The carrier frequency of the fourth stimulation pair of electrodes 4 is 2200 Hz. Hence, the carrier frequencies of the first 1 and second 2 stimulation pairs of electrodes are close one to another, with a difference in frequency of 50 Hz and the carrier frequencies of the third 3 and fourth 4 stimulation pairs of electrodes are close one to another, also with a difference in frequency of 50 Hz. The first 1 and second 2 stimulation pairs of electrodes are thus associated to generate a stimulation in a zone A of the brain that is located in between the pairs 1, 2 of electrodes. In other words, the electric signals provided by the first 1 and the second 2 pairs of electrodes generate a first envelope frequency in a zone A that is equal to the difference between the carrier frequencies of the signals provided by the first and second pair of electrodes 1, 2, i.e. 50 Hz. In the same way, the third 3 and fourth 4 stimulation pairs of electrodes are associated to generate a stimulation in a zone B of the brain that is located in between the stimulation pairs of electrodes 3 and 4. In other words, the electric signals provided by the third 3 and the fourth 4 pair of electrodes generate a second envelope frequency in a zone B that is equal to the difference between the carrier frequencies of the signals provided by the third and fourth pair of electrodes (3,4), i.e. 50 Hz.

The central zone C, that intersects the zones A and B, correspond to the optimal interference area, which is aimed to be stimulated. The stimulation frequency delivered to the optimal interference area C is equal to 50 Hz.

In Fig. 1B, the mean carrier frequency of the first 1 and second 2 stimulation pairs of electrodes is of 1275 Hz. The mean frequency of the third 3 and fourth 4 stimulation pairs of the electrodes is of 2175 Hz. The difference of the mean frequencies of, on one hand, the third and fourth stimulation pairs of electrodes and, on the other hand, the first and the second stimulation pairs of electrodes, is of 2175 - 1275 = 900 Hz. This difference avoids the generation of unwanted stimulation areas at the periphery of the central zone C. A single focal zone is created inside the brain for the stimulation.

On a general point of view, it will be noted that:

advantageously, the carrier frequency of a stimulation pair of electrodes is of at least 800 Hz, preferentially of at least 900 Hz, and more preferentially of at least 1000 Hz;
advantageously, the difference between the carrier frequencies of two stimulating pairs of electrodes that are associated is of at least 25 Hz, but equal or less than 200 Hz. It is for example equal to exactly 50 Hz in Fig. 1B; and
advantageously, in order to avoid unwanted stimulation areas, the difference of the mean frequencies of, on one hand, a first couple of stimulation pairs of electrodes and, on the other hand, a second couple of stimulation pairs of electrodes, is of at least 200 Hz, preferably of at least 500 Hz and even more preferably of at least 800 Hz, for example 900 Hz.

A deep brain stimulation mTI system is also illustrated in Fig. 1C. As appearing on that figure, the system implements stimulation electrodes E in pairs, which are referenced E1A/E1B, E2A/E2B, E3A/E3B and E4A/E4B. There are 4 stimulation electrode pairs defining a quadrupole. These stimulation electrode pairs are positioned on the surface of the scalp, around the head of a mammal, for instance a human individual. The position of the electrodes of a pair of electrodes may be close one to another, even at an immediate vicinity, or at various positions, for instance, at opposite positions around the patient's head. However, the positioning of the electrodes should advantageously be calculated beforehand in such a way as to have a maximum modulation at the point of interest within the brain, while minimizing the electric current delivered by each electrode. If the situation allows it, positioning electrodes on various axes, such as the axes anterior-posterior, medio-lateral or dorsa-ventral, should result in a greater focal gain.

It is also to be noted that the electrodes should not act as capacitors during stimulation in order to avoid non-faradaic processes. Typical Electrocardiogram (ECG) electrodes, which are composed of Ag/AgCl or electrodes coated with Poly(3,4-ethylenedioxythiophene) (PEDOT)-Poly(styrene sulfonate) (PSS) may be used according to the invention.

Any signal generator may be implemented in the system according to the invention, as long as it can generate high frequencies as disclosed herein, and it is possible to modify the phase of the signal. Generally, the generator is connected to one or more electric current sources. The current sources themselves electrically connected to the electrodes. Each pair of electrodes is connected to an individual signal source and the highest stimulation intensity is then concentrated between the electrodes of each such pair. As shown in Fig. 1C, each pair of electrodes is connected to a current source S1 for the first pair of electrodes E1A/E1B, S2 for the second pair of electrodes E2A/E2B, S3 for the third pair of electrodes and S4 for the fourth pair of electrodes. Only the current source S1 is illustrated in Fig. 1C. The current sources are independent. The current source S1 provides a signal function F1 from pair E1. The current source S2 provides a signal source F2 from pair E2. The current source S3 provides a signal function F2 from pair E3 and the current source S4 provide a signal function from pair E4. The signal functions are with corresponding frequencies f1, f2, f3 and f4. The signal function F can be any periodic signal such as sine wave, square wave, monopolar or bipolar pulses, with the frequency f (f1 for F1, f2 for F2, f3 for F3 and f4 for F4.

Focality gain using mTI scales to human brains, but also to smaller animal brains.

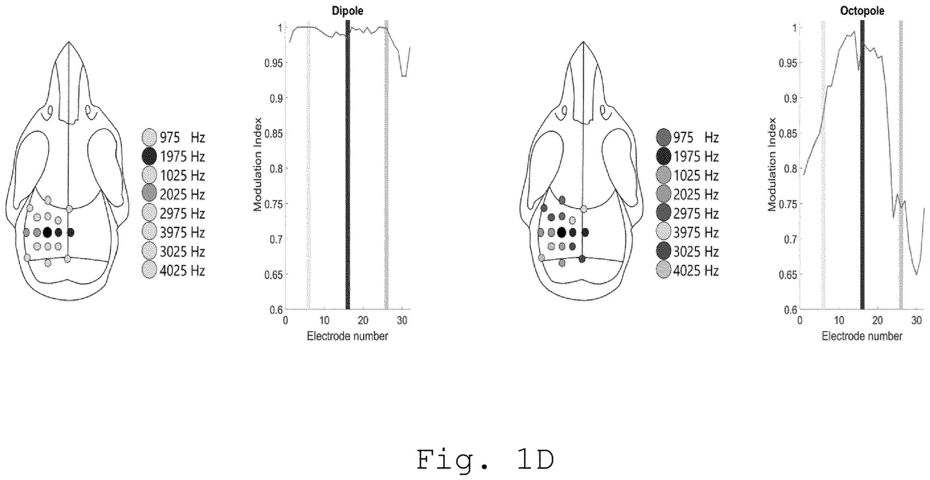

Indeed, as shown in Fig. 1D, standard TI (using only 2 pairs of electrodes) was performed in the mouse brain and compared to mTI, implemented by an octupole, i.e. 8 pairs of electrodes. The envelope amplitude is depicted using the modulation index.

As shown in the left part of Fig. 1D, the two pairs of electrodes provided currents at two frequencies and the envelope amplitude depicted using the modulation index. It is not possible to implant numerous SEEG electrodes in the mouse, therefore a silicon depth probe was implanted through the hippocampal target. The 32 electrodes do not show important variations in modulation index for this electrode configuration, indicating that, for the given exposure configuration, the stimulation reaches the hippocampal target but is not particularly focal. As further seen in Figure 1D, mTI (8 pairs of electrodes providing 8 frequencies generating 4 overlapping envelopes) increases the focality compared to standard TI. Similarly, the focality significantly increases, as evidenced by the reduction of modulation index outside of the hippocampal target (around electrode 16 and below).

On a general point of view, the deep brain stimulation TI system comprises at least two, but advantageously at least four stimulation pairs of electrodes, each stimulation pair of electrodes providing an electric signal at a carrier frequency, the mean value between the carrier frequency of a first stimulation pair of electrodes and the carrier frequency of a second stimulation pair of electrodes defining a first mean carrier frequency, the mean value between the carrier frequency of a third stimulation pair of electrodes and the carrier frequency of a fourth stimulation pair of electrodes defining a second mean carrier frequency, and wherein the difference between said first and second mean carrier frequencies is equal to or greater than 200 Hz.

Preferentially:

- the system comprising between two and a number n of stimulation pairs of electrodes, preferably between four and a number n of stimulation pairs of electrodes, even more preferably height pairs of electrode; the difference between the carrier frequency of a first stimulation pair of electrodes and the carrier frequency of a second stimulation pair of electrodes defining a first envelope frequency; the difference between the carrier frequency of a third stimulation pair of electrodes and the carrier frequency of a fourth stimulation pair of electrodes defining a second envelope frequency; the difference between the carrier frequency of a subsequent stimulation pair of electrodes and the carrier frequency of another subsequent stimulation pair of electrodes defining a subsequent nth envelope frequency, and so on, the values of the first, second, and any subsequent nth envelope frequencies are equal, being especially less than or equal to 500 Hz, being especially equal to between 180 Hz and 1 Hz.
- the carrier frequencies of the stimulation pairs of electrodes are equal or greater than 900 Hz.
- the difference between any two mean carrier frequencies is equal to or greater than 800 Hz, preferably 900 Hz.
- for example, the carrier frequency of a first stimulation pair of electrodes is 1250 Hz, the carrier frequency of a second stimulation pair of electrodes is 1300 Hz, the carrier frequency of a third stimulation pair of electrodes is 2150 Hz, and the carrier frequency of a fourth stimulation pair of electrodes is 2200 Hz.
- the system is configured so that the phase of the envelopes is controlled, especially by using a predefined phase modulation.
- the stimulation amplitude of the electrodes is comprised between 10 $\mu$A and 2500 $\mu$A, better between 1 mA and 2 mA.
- a current is applied to the stimulation pairs of electrodes.
- the system further comprises one or more current sources, the one or more current sources being electrically connected to the pairs of electrodes.

The VEP stimulation workflow

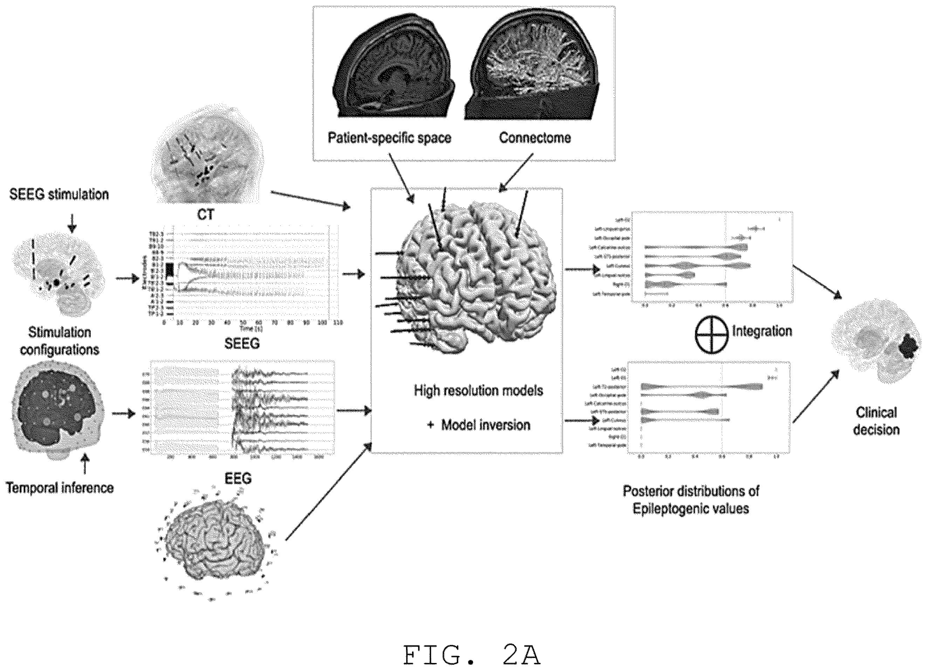

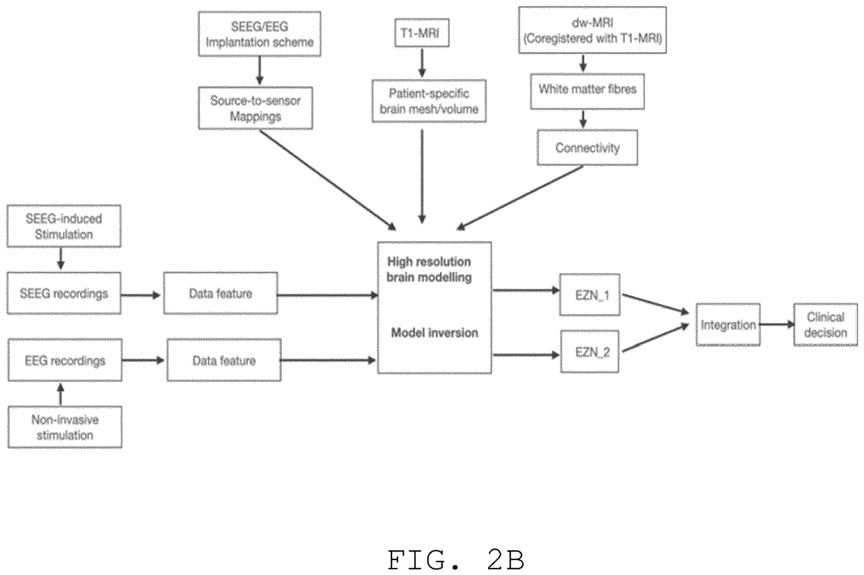

According to the invention, a high-resolution whole-brain modelling workflow is built to estimate the EZ network using stimulation techniques. The EZ network is defined as being the tissue responsible for the generation and spread of epileptic seizures between distant brain areas, and which is characterized by altered excitability and so epileptogenicity. This model-based high-resolution workflow using stimulation techniques for diagnostic EZ mapping is shown in Fig. 2A and the detailed flowchart is shown in Fig. 2B.

As appearing in Fig. 2A, a personalised high-resolution model is based on individual brain structural data, that comprise geometry data extracted from T1-weighted MRI and structural connectivity that are extracted from tractography on diffusion weighted MRI data. This structural connectivity is named the connectome.

Neural field models simulate neural source activity with spatial resolutions of less than 5 mm$^2$, practically of about 1 mm$^2$. Two types of stimulations are used, SEEG and, according to the invention, TI, to simulated seizure induction. The stimulation is non-therapeutic. A post-SEEG-implantation Computed Tomography (CT) scan is used to find exact locations of SEEG electrodes and international standard system 10-5 is for EEG electrodes placement. The gain matrix, which is constructed based on the locations of the SEEG/EEG electrodes, maps the simulated source activity to the corresponding SEEG and EEG signals. Bayesian inference methods are used to estimate the patient-specific parameters of the model based on the data features extracted from the SEEG signals and/or the EEG signals. The output is a posterior distribution of epileptogenic values, which suggest an EZ network candidate.

As appearing in Fig. 2B, first, a T1-MRI defines the patient-specific high-resolution space. Second, the structural connectivity, the gain matrix, i.e. the source-to-sensor matrix, and the sensor-to-source matrix are obtained in a patient-specific brain space by co-registering both diffusion weighted MRI (dw-MRI) and CT for SEEG locations and standard EEG system configuration with T1-MRI. Data features are extracted from the SEEG recordings and EEG recordings from both SEEG-stimulation and TI stimulation and use Hamiltonian Monte Carlo (HMC) techniques for model inversion. From each stimulation module it can be obtained a posterior distribution of epileptogenic values, which suggest EZ network candidates. Also it is introduced the integration module to combine multiple results in different cases to help the clinical decision.

First, high-resolution full brain network models are established using patient-specific data in epilepsy. The structure of the model is provided by the detailed surface of the cortex and subcortical structures as well as the heterogenous structural connectivity between brain regions through white matter fibers as estimated from dw-MRI. The cortical surface data is generated from T1-weighted MRI using the Freesurfer™ software package, resulting in surfaces with ~260,000 vertices per hemisphere, with a vertex area of ~1 mm². Seizure is simulated like dynamics on the patient specific structural scaffold using the phenomenological model named Epileptor™ disclosed in the document Jirsa VK, Stacey WC, Quilichini PP, Ivanov AI, Bernard C. 2014, "On the nature of seizure dynamics", Brain 137:2210-2230, a system of differential equations that describes seizure initiation, propagation, and termination, resulting in realistic electrophysiological seizure like events. The spatial domain of the Epileptor are the cortical neural fields and subcortical neural masses, thus seizures can propagate locally across neighboring vertices of the cortex and globally through white matter fiber connections. A post-SEEG implantation CT scan is used to localize SEEG contacts and co-register them with the structural scaffold. For EEGs, the standard international 10-5 system is used to place EEG electrodes. The high-resolution model allows to consider detailed electric dipoles, generated by neural activity, for building high-fidelity forward solutions. The gain matrix maps activity from neural sources - the brain vertices - to electrodes SEEG/EEG, based on their orientation and distance to each other.

The two types of stimulation techniques are included into the modelling approach for diagnostic EZ network mapping. First, the high-resolution model is built for SEEG stimulation, where the stimulation is induced through SEEG electrodes. The stimulation is applied on the vertices of the high-resolution surface through the SEEG-to-source mapping. To simulate TI, the intensity of the TI field is first computed on a patient's brain, and then integrated into the whole brain model, to investigate the capability of TI to trigger seizures. Stimulation leads to an accumulation on a phenomenological slow-evolving variable m in the Epileptor™ stimulation model that is disclosed below. When, in the Epileptor model, m reaches a defined threshold, it can push the model into the seizure state, causing fast discharges on the other state variables of the model.

Model inversion infers the patient-specific brain model parameters, especially epileptogenicity and the global coupling scale working on structural connectivity. The model inversion module employs HMC sampling techniques from Bayesian inference methods to estimate a patient's EZ network. The Bayesian inference methods output the posterior distribution of key parameters of personalised models based on the data feature and prior. The data features use SEEG and/or EEG signals. The output of the model inversion module are the posterior distributions of epileptogenic values, which leads to the predicted EZ network. The integration module is also introduced to combine the results of multiple SEEG and EEG signals, and the HMC integration model inversion on the simultaneous SEEG/EEG is implemented, which will aid the final clinical decision.

VEP SEEG-induced stimulation

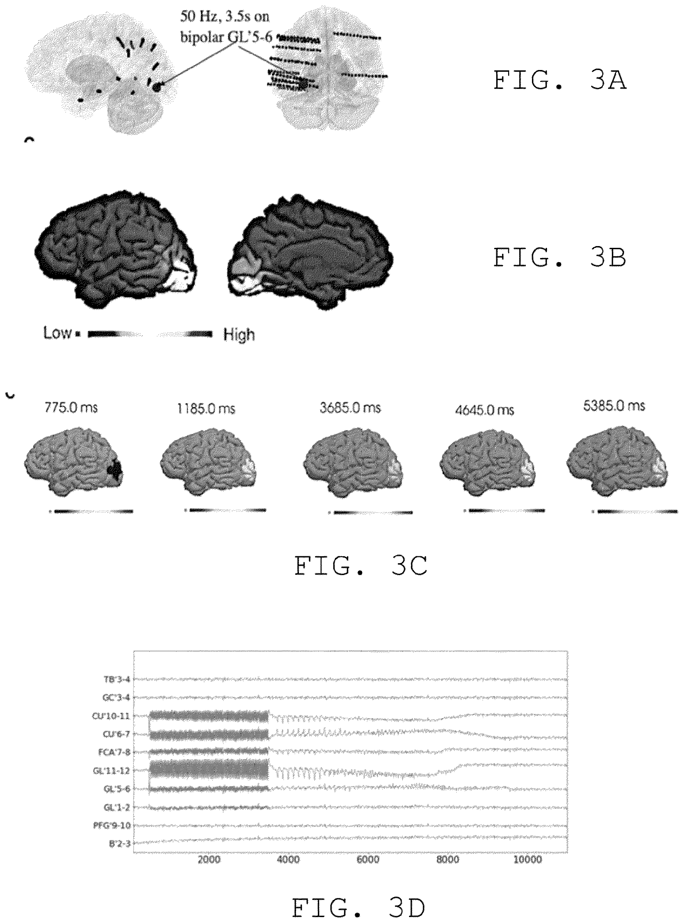

An example of the two types of stimulation techniques is provided using one patient, initially diagnosed with left occipital lobe epilepsy. The structural connectivity matrix is first extracted and source-to-sensor mapping from individual T1-MRI, dw-MRI, and post-SEEG-implantation CT imaging data. To model brain stimulation from one pair of SEEG electrode leads, the contribution is retrieved from a pair of electrodes where the stimulation is being applied to the brain regions from the sensor-to-source mapping matrix. Then this is further used to map the effect of the bipolar pulse stimulation (50 Hz, 3.5s on bipolar GL'5-6 electrode leads) on each vertex of the neural field cortex mesh (Fig. 3A). Combined with the temporal stimulation waveform, a spatio-temporal representation of the bipolar pulse stimulation is obtained from a pair of SEEG electrodes on the whole brain (Fig. 3B). The Epileptor stimulation model is used on each of the 260,000 vertices representing the cortical mesh. The stimulation of the Epileptor leads to an accumulation in a state variable m finally causing the brain to seize. The seizures are located around the left-O2 regions in VEP atlas.

From these simulated neural sources (Fig. 3C), SEEG signals (Fig. 3D) are obtained using the source-to-sensor gain matrix.

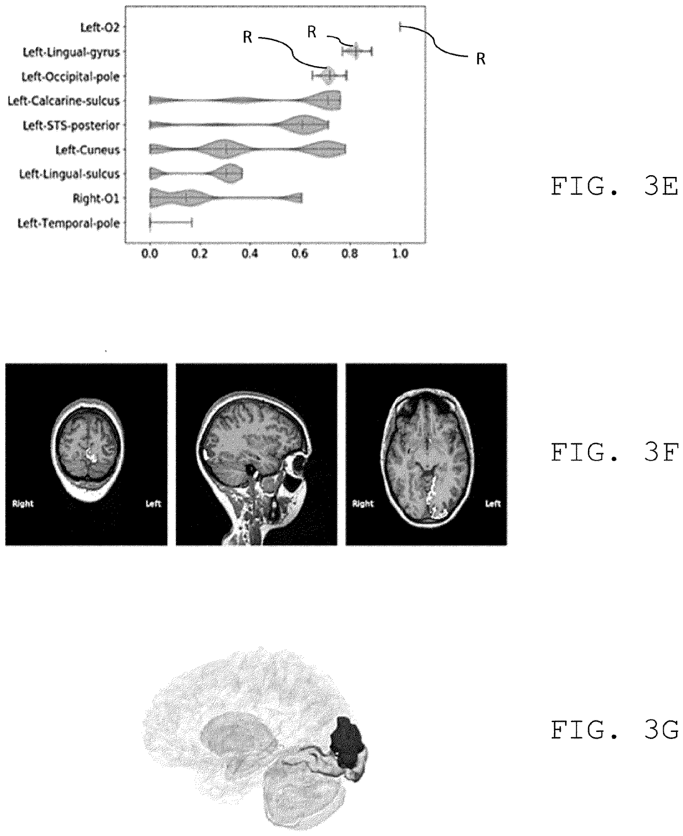

Hence, in Fig. 3A, stimulated contact is GL'5-6 (large sphere) in the left occipital lobe, using a bipolar pulse stimulation (50Hz for 3.5s). In Fig. 3B, the spatial mapping of the maximum of stimulation values (shown in the bar from low (left) to high (right) values) to each vertex of the brain from sensor GL'5-6. In Fig. 3C, the neural activity is shown on the cortical mesh at 5 different time points. The seizures are located around the left-O2 region of the VEP atlas. In Fig. 3D, selected simulated SEEG time-series from SEEG stimulation induced seizure of the example patient. In Fig. 3E, posterior distribution of the epileptogenic values (EV) (higher value indicates higher chance for seizure) of 9 selected regions obtained from HMC sampling. Regions R indicate highest chance to be EZ network. The region of the highest EV posterior distribution is Left O2 in pale gray shown in T1-MRI (Fig. 3F) and shown in dark gray in the 3D brain illustration in Fig. 3G. The two regions (left lingual gyrus and left occipital pole) are shown in very pale gray in Figs. 3F, 3G.

The synthetic SEEG signals (Fig. 3D) are used as an input to the VEP pipeline to estimate the EZ network. The posterior distribution of the EVs from the sampling pipeline is based on 16 chains starting from 8 different optimized initial conditions. Each HMC chain creates 500 samples. The Left-O2 with highest epileptogenic values is obtained from all samples (Fig. 3E). The two neighboring regions (Left-Lingual-gyrus and Left-Occipital-pole) are the second-group candidates belonging to the EZ network. The heatmap projected on the patient's T1-MRI shows the spatial mapping of the EZ network (sagittal, axial and coronal view images shown in Fig. 3F). The spatial mapping of these three regions in 3D brain is shown in Fig. 3G.

## VEP TI stimulation

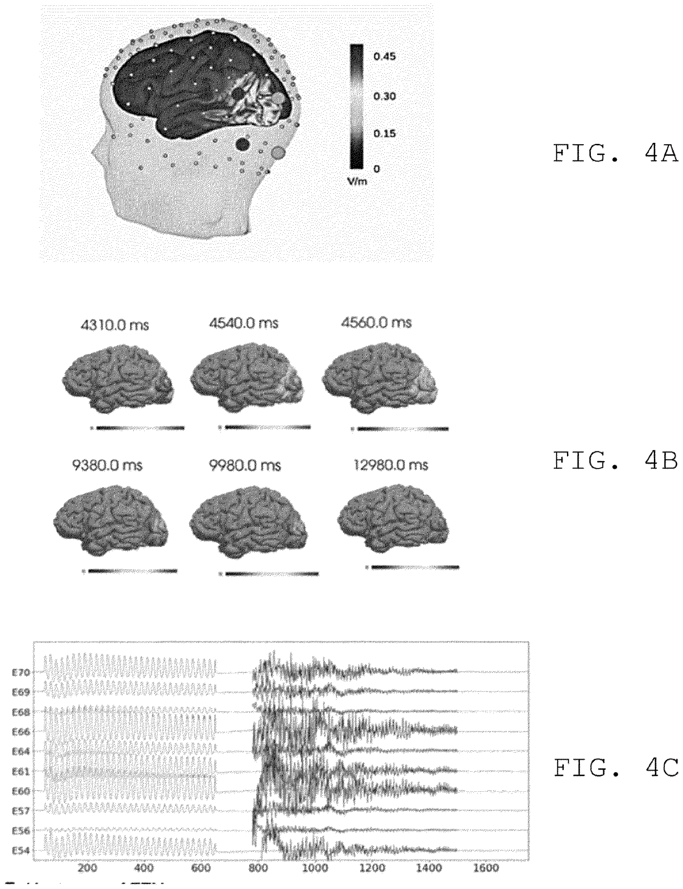

Neural field high resolution modelling is used to model TI stimulation. First, the TI field is calculated, which is projected onto the cortical surface using the normal vector to the surface as unit vector (Fig. 4A). This preferential direction is chosen given the assumption that the components of the field, that have the greatest impact, are those that are parallel to the neuron's axons. Then, the resulting time-resolved scalar values of the TI field is used as input to the Epileptor model in each vertex of the cortical mesh. The brain signals are simulated on each vertex of the whole brain in neural field level (Fig. 4B). The generated seizure is then mapped from signals of each vertex in the source space onto EEG sensors (Fig. 4C) using the source-to-sensor mapping matrix.

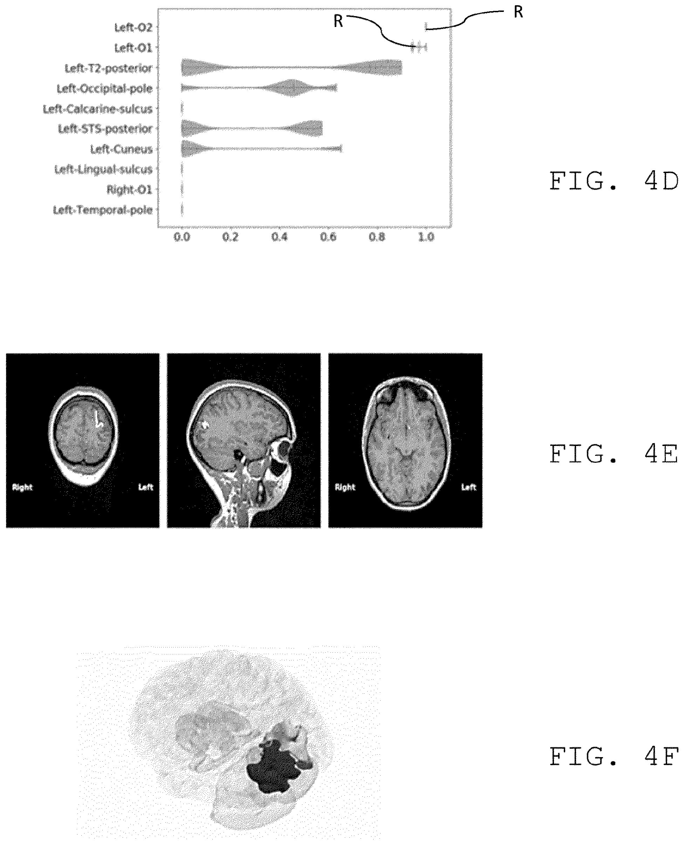

Synthetic EEG recordings as shown in Fig. 4C are used as input to the VEP pipeline to estimate the epileptogenic zone. The model inversion used a non-informative prior in which all the brain regions followed the same prior distribution. The posterior distribution of EVs from the HMC sampling was based on 16 chains starting from 8 different optimized initial conditions. Each HMC chain created 500 samples. The left O2 is obtained with highest epileptogenic values from all samples and the left O1 as the second candidate belonging to the EZN (Fig. 4D). The heatmap of these two regions projected on the patient's T1-MRI shows the spatial mapping of the EZ network (sagittal, axial and coronal view images shown in Fig. 4E) and 3D brain in Fig. 4F.

Hence, in Fig. 4A, the electric field of TI stimulation by two pairs of EEG electrodes (illustrated by black and gray circles) based on the 10-5 international reference system. It is applied a frequency of 1000 and 1005 Hz in the first and second electrode pairs, respectively. Spatial distribution of the peak activity of the TI envelope are colored in dark gray in 3D brain. In Fig. 4B, seizure dynamics is simulated using the stimulation Epileptor through the TI stimulation. Neural activity is shown on the cortical mesh at 6 different time points. The seizures are located around the left-O2 regions of the VEP atlas. In Fig. 4C, selected simulated EEG time-series from TI stimulation induced seizure. The scaled-up time series during the seizure period are shown in dark gray. In Fig. 4D, posterior distribution of the epileptogenic values (EV) (higher value indicates higher chance for seizure) for 10 selected regions obtained from the MCMC sampling. Regions R indicate highest chance to be EZ network. The dashed vertical line indicates the seizure threshold. The region of the highest EV posterior distribution is Left O2 shown in T1-MRI (Fig. 4D) and shown in black in the 3D brain illustration in Fig. 4F. The second region (Left O1) are shown in white in Figs. 4E, 4F.

## Integration module

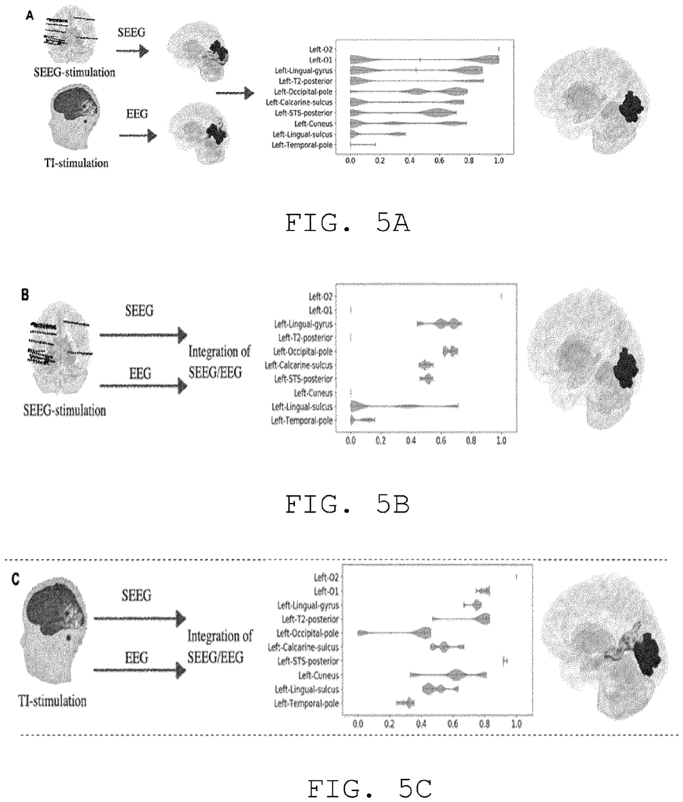

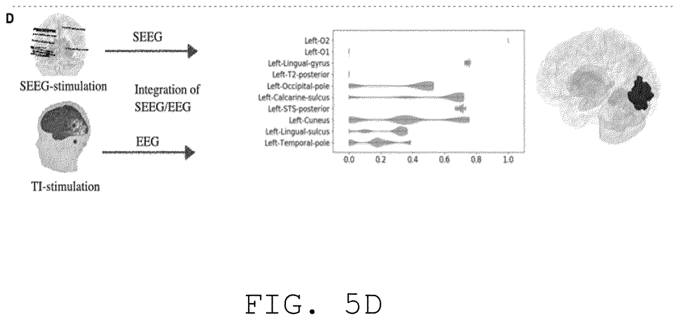

For patients with multiple seizure recordings or simultaneous multiple recordings, an integration module is introduced to provide more information for clinical decision. The integration module provides four possible approaches to integrate EZ network estimation from multimodal functional recordings, such as EEG and SEEG. The first approach is to pool the posterior distribution of results from SEEG (in Fig. 3E under SEEG stimulation) and EEG (in Fig. 3D under TI stimulation). Then, left O2 is obtained as intersection of the two distribution and the only converged brain regions for EZ network (Fig. 5A). The second approach is to estimate the EZ network from simultaneous EEG and SEEG recordings under SEEG stimulation. Here, the same source signals (Figure 3B) are mapped to both SEEG and EEG signals in both simulation module and model inversion module. This version is called as the simultaneous model inversion. 16 chains are ran from 8 different optimized initial conditions. The posterior of EV from simultaneous model inversion shows left O2 as the EZ (Fig. 5B). The third approach is to estimate the EZ network from simultaneous EEG and SEEG recordings under TI stimulation. The posterior of EV from HMC model inversion shows left O2 as the EZ and left STS-posterior as a second candidate (Fig. 5C). In the fourth approach, the same data are worked out as the first approaches in Fig. 5A but the simultaneous model inversion as the second and third approaches in Figs. 5B, 5C. The posterior of EV from simultaneous model inversion shows Left-O2 as the EZ (Fig. 5D). It is noticed that the functional SEEG and EEG data here are not simultaneous recordings. The results would be valid only with an assumption that HMC model inversion sampled all feasible initial conditions. For this simple patient example, the integrated results add the confident values for left-O2 as the EZ.

Hence, in Fig. 5A, a combination of the posterior distribution of EVs by pooling the posterior distribution obtained from SEEG (Fig. 3E under SEEG stimulation) and EEG recordings (Fig. 3D under TI stimulation). In Fig. 5B-5D, the posterior distribution of EVs from HMC based on the whole brain models which simultaneously forward to SEEG and EEG. These simultaneously can be performed in three cases: seizures induced by SEEG stimulation (Fig. 5B), TI (Fig. 5C) and both SEEG and TI (Fig. 5D). Each row shows the data used for model inversion, the posterior of EVs and the corresponding highlighted EZNs. In the 3D brain, the left-O2 is in black and left STS-posterior is in white.

Finally, the invention provides a dedicated stimulation diagnostic pipeline based on the whole brain modelling for epilepsy. This pipeline is validated on simulated data from high resolution neural field modelling. The estimation of the EZ network as output of model inversion is performed by HMC of the Bayesian inference. It is demonstrated the estimation of EZ network from SEEG and EEG signals under the SEEG and TI stimulation. Two model inversion versions are introduced, one with single functional data such as SEEG or EEG, and one with simultaneous SEEG and EEG data. The introduced stimulation pipeline provides a methodological and conceptual basis for further scientific studies and clinical translations.

The first application of this pipeline answers an important diagnostic question: can we infer the underlying EZ networks from stimulated seizures? To answer this question, it is needed to investigate the following questions. First, what is the proper range of stimulation parameter to induce seizures for diagnosis? Currently standardized stimulation protocols do not exist. The French guidelines suggest parameters for both low-frequency (1Hz) and high-frequency (50Hz) with specified ranges on pulse width, pulse intensity and stimulation. The whole brain modelling can help to predict the stimulation location of electrodes for given patients. Second, on the patient-specific level, what are the optimal stimulation parameters to induce seizures for diagnosis? And then third, what are the sufficient induced seizures by stimulation for identifying the full underlying EZ network? More specifically, do the induced seizures introduce the artificial EZ network or create the larger EZ network due to the neural modulation? Comparison with spontaneous seizures can be a side approach, but the limited observable recorded spontaneous seizures may only tell the partial stories. To answer all these questions, this introduced pipeline with the high-resolution modelling provide a reasonable framework.

A digital brain twin or a useful virtual epilepsy patient is built, which requires to integrate the multimodal neuroimaging, such as anatomically recordings like TI weighted MRI, diffusion weighted MRI, CT, DTI and PET, and functional ones like EEG, MEG, SEEG, SDG and fMRI. The introduced framework according to the invention moves forward on this direction and the integration module showed the possible approaches to integrate information from EEG and SEEG, which can be easily extended to other recordings, such as MEG, fMRI and SDG, depending on the data availability. The only difference is the forward solution matrix from different measurement. It is designed an integration version of HMC algorithm for the simultaneous functional recordings such as SEEG/EEG or MEG/EEG or SEEG/MEG. The proposed pipeline also severed a basic structure to estimate EZ network during interictal spike from SEEG/MEG. The usage of this simultaneous version is also valid in the cases where different recordings start with same brain states, or the sample algorithms can cover all possible initial conditions.

To introduce the diagnostic pipeline for TI stimulation, the invention aims to provide a theorical basis for the non-invasive estimation of the location of epileptogenic zone networks. TI stimulation is a good candidate for diagnosis in epilepsy within the non-invasive stimulation family, which others, such as tDCS, is mostly used only for treatment in epilepsy. The key features of TI stimulation enable focused yet reach the deep brain structures, such as hippocampus, which quite often is involved in the EZ network in epilepsy. TI stimulation evoked seizure-like events in the mouse hippocampus have been experimentally well controlled. The invention provides the pipeline to predict the usage of diagnosis EZ network for human epilepsy and as a basis for optimization strategy for stimulation parameters. Then, based on this provided framework, it is possible to build high resolution modelling for individual patients in the future therapeutic intervention. Although, the example of patients with EZ in the cortical region works fine with EEG signals for non-invasive measurement. The current EEG system cannot effectively measure the signals from the deep structures.

The current pipeline has its limitations. Model-based approaches are usually computationally expensive and parameter sensitive, which may pose challenges for real-time use in clinical routines such as stimulation parameters optimization. The current pipeline doesn't consider the dynamic of epileptic disorder over long timescales or the long-time effect of the stimulation. The ongoing systematic evaluation and studies will improve the current pipeline in the following aspects. The high-resolution modelling in subcortical structure and more realistic forward resolution may improve the whole brain modelling. The high-resolution model inversion will be able to add more values in the high-resolution modelling. The introduction of region variance (such as cell density and receptor density) may provide a fundamental way to improve the current VEP pipeline.

### Materials and methods

### Patient data

This patient is a right-handed 23-year-old female initially diagnosed with left occipital-temporal epilepsy. This patient underwent a standard presurgical protocol at La Timone Hospital in Marseille, France. This patient underwent comprehensive presurgical assessment, which includes medical history, neurological examination, neuropsychological assessment, fluorodeoxyglucose positron emission tomography, high-resolution 3T MRI, long-term scalp EEG and invasive SEEG recordings. The patient has non-invasive T1-weighted imaging (MPRAGE sequence, repetition time = 1.9 or 2.3 s, echo time = 2.19 or 2.98 ms, voxel size $1.0 \times 1.0 \times 1.0$ mm$^3$) and diffusion MRI images (DTI-MR sequence, either with an angular gradient set of 64 directions, repetition time = 10.7 s, echo time = 95 ms, voxel size $1.95 \times 1.95 \times 2.0$ mm$^3$, b-weighting of 1000 s $\times$ mm$^2$, or with an angular gradient set of 200 directions, repetition time = 3 s, echo time = 88 ms, voxel size $2.0 \times 2.0 \times 2.0$ mm, b-weighting of 1800 s/mm$^2$. The images were acquired on a Siemens™ Magnetom Verio™ 3T MR-scanner. The patient had invasive stereo-EEG (SEEG) recordings obtained by implanting multiple depth electrodes, each containing 10-18 contacts 2 mm long and separated by 1.5 or 5 mm gaps.

The SEEG signals were acquired on a 128 channel Deltamed/Natus system. After the electrode implantation, a cranial CT scan was performed to obtain the location of the implanted electrodes.

### Data processing

To construct the individual brain network models, the T1-MRI and DW-MRI data were first preprocessed. Volumetric segmentation and cortical surface reconstruction were handled from the patient-specific T1-MRI data using the recon-all pipeline of the FreeSurfer™ software package. The cortical surface was parcellated according to the VEP atlas. The MRtrix™ software package was used to process the DW-MRI, employing an iterative algorithm to estimate the response functions and subsequently used constrained spherical deconvolution to derive the fiber orientation distribution functions. The iFOD2 algorithm was used to sample 15 million tracts. The structural connectome was constructed by assigning and counting the streamlines to and from each VEP brain region. The diagonal entries of the connectome matrix were set to 0 to exclude self-connections within areas and the matrix was normalized so that the maximum value was equal to one. The location of the SEEG contacts from post-implantation CT scans were obtained using the Graphical user interface for Automatic Registration and Depth Electrodes Localization (GARDEL), as part of a software package named EpiTools™. Then, the contact positions from the CT scan space to the T1-MRI scan space of each patient were co-registered.

### Calculation of the electric field of TI stimulation

To obtain the distribution of the TI field in the gray matter, it was started from the computation of astatic electric field distribution resulting from the simulation of a transcranial Alternating Current Stimulation (tACS) via the software SimNIBS™. First, the individual T1-weighted MRI was reprocessed via the SIMNIBS process "mri2mesh", which resulted in the segmentation in 5 tissues: CerebroSpinal Fluid (CSF), White Matter (WM), Gray Matter (GM), skull and scalp. This process creates the triangular and tetrahedral meshes, which is used for the Finite Element Method (FEM) simulation of the electric field distribution. The tACS field was computed for each of the two electrode couples positioned based on the location of the co-registered 64 electrodes EEG cap, using the 10-5 system. Here, it was used, for the simulation, circular electrodes of a diameter of 10 mm and a thickness of 5 mm directly applied on the head. Second, these fields were projected on the resampled Freesurfer™ surface (20484 vertices) obtained from the previous pre-processing pipeline. Specifically, it was extracted the scalar value of the norm E, obtaining in this way a value of the static electric field for each vertex of the cortex surface. These fields derived from the two electrodes couples were then modulated by multiplying them with two sinusoids with the frequency of 1000 Hz and 1005 Hz, respectively. To obtain a correct representation of the high frequency activity, the sampling rate was kept at 30kHz, with a signal length of 1 sec. This resulted in two oscillatory electric fields, which were linearly summed, obtaining in this case an interfering field. Finally, the 5 Hz peak envelope was extracted by using spline interpolation over local maxima separated by a 100 number of samples. Via this analysis pipeline, it was obtained a 5Hz oscillating envelope, which amplitude varied amongst the cortex vertices (20484). Finally, this envelope was included in the neural field model as source of external simulation.

For this patient, the region left-O2 was targeted in VEP atlas. To stimulate this region, the optimization process included in SIMNIBS was used. This procedure results in the identification of the best 4 electrodes configuration (10-5 EEG montage) to maximize the electric field in left-O2. The best configuration was defined here as the one giving us the most focal activation. This procedure allowed to identify PPO3-PPO5 and PSh-POSh as the best electrodes couple for the left-O2 stimulation.

High resolution modeling for stimulation

The high-resolution brain model was built. The activity of each vertex of brain meshes was modelled with a set of dynamical equations. For simulating the seizure activity, the original Epileptor model was extended for the Epileptor-stimulation version as follows:

$$\dot{x}_1 \;=\; y_1 - f_1(x_1) - z + I_{ext1} + I_{stim}$$

$$\dot{y}_1 \;=\; c - dx_1^2 - y_1$$

$$\dot{z} \;=\; r(4(x_1 - x_0 + \mathrm{nH}(\mathrm{m} - m_{thresh})) - z) + f_3(z) + K\sum_{j=1}^{N} C_{i,j}\,(x_1^j - x_1^i)$$

$$\dot{m} \;=\; r(k|I_{stim}| - m)$$

where

$$f_1(x_1) \;=\; \begin{cases} ax_1^3 - bx_1^2 & \text{if } x_1 < 0 \\ -(m + 0.6(z-4)^2)x_1 & \text{if } x_1 \geq 0 \end{cases}$$

$$f_3(z) \;=\; \begin{cases} -0.1z^7 & \text{if } z < 0 \\ 0 & \text{if } z \geq 0 \end{cases}$$

*The state variables $x_1$ and $y_1$ describe the activity of neural populations on a fast time scale and can model fast discharges. The oscillation of the slow permittivity variable z drives the system autonomously between ictal and interictal states. The parameter $x_0$ indicates the degree of excitability and directly controls the dynamics of the neural population to produce the seizure or not. The default parameters are $I_{ext1}$ = 3.1, c = 1, d = 5, $\tau$ = 10, r = 1, K = -5, a = 1, m = 1. The coupling between nodes of the network is defined by $C_{i,j}$, which comes from the structural connectivity. K scales the connectivity. For stimulation effects, parameter $I_{stim}$ and parameter m were introduced as variable.*

To reproduce the theories of reduced resilience of the epileptogenic zone and an increase in spike frequency and oscillations when an external perturbation was applied in the Epileptor model, the relevant parameters were studied, that influence the oscillatory activity of the upstate, also known as the seizure state, in particular, the parameter m. It was hypothesized that, for stimulation induced seizure, a build-up of ion imbalances reaches a seizure threshold followed by an epileptic seizure. The equations were modified by converting the phenomenological m parameter to a variable, which accumulates the effects of the external stimulus until the seizure threshold is reached.

To model the effects of any external stimulation, first it is needed to compute the generated electric field from the stimulation device. Once the stimulation field has been computed, it is injected as $I_{stim}$ parameter in the model. This method is the same for any type of stimulation. It is here demonstrate it on two types of stimulation: a) an invasive SEEG stimulation, b) a non-invasive brain stimulation with TI according to the invention.

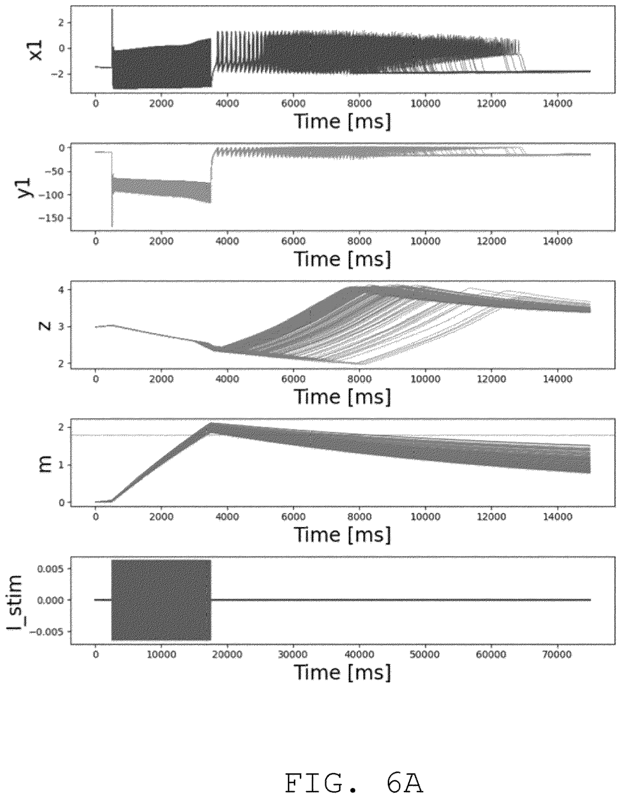

The SEEG stimulation is applied on a pair of neighboring sensors, in which one acts as a cathode and the other one as an anode. This generates a bipolar pulse stimulation in the area where the electrodes are located. However, the effects of the stimuli are not only local, but they can propagate through short-range and long-range connections towards other brain areas. The parameters used clinically are restricted to frequencies of either 1Hz or 50Hz, weak amplitudes ranging from 0.1 to 3 mA and pulse width of 500 - 2000 microseconds. It was modelled the same stimulus applied clinically on a personalised digital brain model of the patient. Using the reconstructed brain structure and the overlayed SEEG electrodes, it was generated a bipolar signal using the stimulation parameters in the pair of electrodes chosen by the clinician. The stimulus signal was then mapped onto the parcellated brain areas based on the distance, which results in an estimated electric field on the brain regions. This estimated field was used as an input to $I_{stim}$ parameter

and simulated time series on one selected vertex from EZ are shown in Fig. 6A.

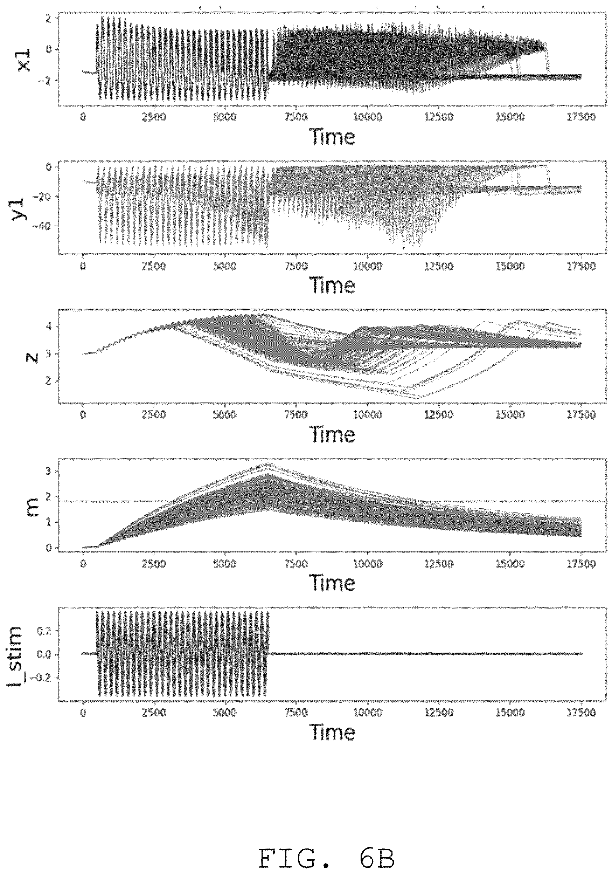

Non-invasive stimulation with TI is a recent method which aims to mimic DBS's capabilities of being focal and subcortical without being invasive. This is done using the assumption that high frequencies, above 900Hz, have ignorable effect on neuronal activity. By combining multiple fields of very high-frequency tACS stimulation, such that they interfere maximally in the target of choice, a new electric field is generated which modulates at much lower frequencies. For example, two fields of 1000Hz and 1050Hz modulate at 50Hz at the target of choice. The generated electric field is computed using SimNIBS™ as described previously, in order to compute the generated tACS fields. Then, the vectorial tACS fields are added together, which results in a TI field estimation on the brain regions. This estimated field was used as an input to $I_{stim}$ parameter and simulated time series on one selected vertex from EZ are shown in Fig. 6B.

Hence, in Fig. 6A, SEEG-stimulation and, in Fig. 6B, TI. Five state variables of the model at seizing parts of the cortex and the stimulation time course (I_stim). The stimulation is accumulating on state variable m of the Epileptor. Once m passes a fixed threshold it causes the model to initiate the seizure.

Whole brain network modelling

This relates two types of whole brain modelling: neural mass modelling and neural field modelling. Neural masses represent the macroscopic behavior of populations of neurons. They are defined by a set of differential equations that govern their dynamics. In the Virtual Brain, neural masses represent the activity of a single brain area. Neural masses are linked through the structural connectome to form a full brain network model. The global equation for such a model can be given by

$$\dot{\psi}_i(t) = F(\psi_i(t)) + K \sum_{j=1}^{L} W(i,j) S(\psi_i(t), \psi_j(t))$$

where $\psi_i(t)$ is a state vector of neural activity at brain region $i$ and time $t$. $\dot{\psi}$ is the temporal derivative of the state vector. $F$ is a function of the state and captures the local neural activity. In our case, $F$ reflects the Epileptor model, described above. $W$ is a matrix of heterogeneous connection strengths between node $i$ and $j$. $S$ is a coupling function of the local state $\psi_i$ and the distant delayed state $\psi_j$. That a node receives input through the network is given by the sum across the number of nodes $L$ and scaled by a constant $K$. In this paper, this set of differential equations is solved using an Euler integration scheme with a step size of 0.5 ms in the virtual surgery simulations.

Neural fields extend neural masses along the spatial dimension. Instead of accumulating neural activity in a single point, the activity is represented continuously across the cortical surface. The global equations for such a model can be written as

$$\dot{\psi}_i(x,t) = F(\psi_i(x,t)) + K_{het} \sum_{j=1}^{L} W_{het}(i,j) S_{het}(\Psi_i(t), \Psi_j(t))$$

$$+ K_{hom} \int_{\Gamma} W_{hom}(|x - y|) S_{hom}(\psi_i(x,t), \psi(y,t))\, dy$$

The spatial dimension of the field is given by two closed surfaces representing the pial cortex, each for one hemisphere. The surface is discretized by ~260,000 vertices. The neural state of a vertex $i$ at position $x$ and time $t$ is given by $\psi_i(x,t)$. The subscript $i$ indicates the brain region to which the vertex is assigned. There are 2 types of coupling in this model. The heterogeneous coupling through $W_{het}$ and $S_{het}$ is similar to the previous NMM approach, but the input to the coupling function $S_{het}$ now is $\Psi_i$ and $\Psi_j$, the average neural state of all vertices belonging to regions $i$ and $j$, respectively. The homogeneous coupling is given by the integral across the spatial domain $\Gamma$. $W_{hom}$ is a translation-invariant coupling strength function based on the geodesic distance between vertices located at position $x$ and $y$ along the surface. In the implementation, this function is described by $W_{hom}(x) = \frac{1}{2} e^{-|x|}$. $S_{hom}$ is the coupling function of the neural state $\psi_i$ at local position $x$ and time $t$ and the neural state $\psi$ at position y and time t, regardless of the region assignment of the vertices. $K_{het}$ and $K_{hom}$ are the scaling parameters for heterogeneous and homogeneous couplings, respectively. Including both couplings with the increase in spatial resolution of the model from 162 brain regions in NMM to ~260000 vertices in MFM causes greatly increased computational demand.

Forward solution for SEEG signals

Mapping the neural activity from the sources (VEP brain regions in NMM and vertices in NFM) to the sensors (SEEG contacts) is done by solving the forward problem and estimating a source-to-sensor matrix (gain matrix). As sources for our model, the vertices of the pial surface and volume bounding surfaces for the cortical and subcortical regions were used, respectively. Surfaces are represented as triangular meshes. For the NMM approach it is estimated that the matrix $g_{j,k}$ from source brain region $j$ to sensor $k$ is equal to the sum of the inverse of the squared Euclidean distance $d_{i,k}$ from vertex $i$ to sensor $k$, weighted by the area $a_i$ of the vertex on the surface.

$$g_{j,k} = \sum_{i=0}^{N_j} \frac{a_i}{d_{i,k}^2}$$

Here vertex $i$ belongs to region $j$ which has $N_j$ vertices in total. The area $a_i$ of vertex $i$ is obtained by summing up one-third of the area of all the neighboring triangles. Vertices belonging to the same brain region are summed to obtain the gain for a single region of our brain network model. The resulting gain matrix has dimensions $M \times N$, with $M$ being the number of regions and $N$ the number of sensors. Matrix multiplication of the simulated source activity with the gain matrix yields the simulated SEEG signals, i.e. $SEEG_k(t) = \Sigma_j g_{j,k} x_j(t)$, where $x_j(t)$ is the time series of source level signals.

This distance-based approach and the summation of all vertices within each region neglects the orientation of the underlying current dipoles. Pyramidal neurons, which are oriented normal to the cortical surface, are assumed to be the physiological source of any electrical signal recorded with SEEG, EEG, or MEG. This could not be taken into account when modeling a brain region by an NMM. However, approaches that use neural fields have this ability. To solve the forward problem an analytical solution was followed for electrical fields in an unbounded homogeneous medium. Possible boundary effects were neglected because SEEG records within the brain where electrical conductivity is almost constant. Therefore, the gain matrix elements $g_{i,k}$ can be estimated for the NFM approach by

$$g_{i,k} = \frac{a_i}{4\pi\sigma} Q * \frac{r_k - r_i}{|r_k - r_i|^3}$$

where $r_k$ and $r_i$ are the position vectors of sensor $k$ and source vertex $i$, respectively. $|v|$ represents the L2 norm of a vector $v$. $Q$ is the dipole orientation vector and $\sigma$ the electrical conductivity. Since it is assumed constant conductivity across the brain, it becomes merely a scaling factor, which we set to $\sigma = 1$.

Forward solution for EEG signals

To compute the forward solution of EEG signals, three individual surfaces (i.e. inner skull, outer skull and head) of the patient based on Boundary Element Models (BEM) using Brainstorm were first reconstructed. Then we co-registrated of EEG electrodes (in Brainstorm) by projecting the EEG sensor positions of the Hydrocel E1 128 channels electrode cap, on the head surface according to the fiducial points of the patient's T1-MRI. It was applied slight manual correction to better orient the EEG cap on the individual anatomy. Finally, an EEG forward model was derived using 3-shell BEM model (conductivity: 0.33, 0.165, 0.33 S/m; ratio: 1/20) and OpenMEEG method implemented in Brainstorm, which provides a realistic head model. Then, it was obtained a gain value for each dipole (20484 vertices), with the constrained direction normal to the cortex surface, on each EEG electrode. Finally, the gain matrix derived from the head model was multiplied with the simulated time series of the brain sources to obtain the *in silico* scalp EEG activity,

i.e. $EEG_l(t) = \Sigma_j g_{l,j}^{EEG} x_j(t)$,   $g_{l,j}^{EEG}$ from source brain region $j$ to EEG $l$.

Calculate the SEEG/EEG data features

The data feature extraction was extracted from the SEEG and EEG signal to be the input of the model inversion modules. The SEEG data is re-referenced using a bipolar montage, which is obtained using the difference between 2 neighboring contacts on one electrode. The 2D Epileptor model is suited to fit the envelope of the seizure time series. Ideally, the envelope follows a slightly smoothed rectangular function from the onset till the offset of the seizure. To get a well-formed target our model should fit, the bipolarized SEEG signal was extracted from 5s to 15s before seizure onset until 5s to 15s after seizure offset. It was identified outlier time points which are greater than 2-times the standard deviation of the extracted signal and replace them with the mean of the extracted signal. The signal is then high-pass filtered with a cut-off at 5Hz to 30Hz to remove slow signal drifts, with the default value of 10Hz. The ideal cut-off depends on the different epileptiform activities in order to extract a well-formed envelope. The envelope is calculated using a sliding-window approach with a window length of 100 time points. The signal inside the window is squared, averaged and log transformed. From the resulting envelope we again identify and remove outliers as described above. Finally, the envelope is smoothed using a lowpass filter with a cut-off in the range of 0.02Hz to 1 Hz, with default value of 0.07Hz. The mean across the first few seconds of the envelope is used to calculate a baseline which is then subtracted from the envelope.

The HMC model inversion

Taking advantage of time scale separation and using averaging methods, the Epileptor is reduced to a 2D system:

$$\dot{x}_i = I_1 - x_i^3 - 2x_i^2 - z_i$$

$$\dot{z}_i = (1/\tau_0)(4(x_i - x_{i,0}) - z_i + K \sum_{j=1}^{N} C_{i,j} (x_j - x_i))$$

where $\tau_0$ scales the length of the seizure. The external input is defined as $I_1$ = 3.1. The 2D Epileptor was used for model inversion and estimated the parameters of the model from empirical data. Then, the source level signal was mapped to EEG and SEEG signals. For simultaneous model inversion, the same source signals was sampled to the EEG and SEEG at the same time.

For the model inversion, it was applied the No-U-Turn Sampler (NUTS), a self-turning variant of the HMC algorithm to sample the posterior density $p(\theta|y)$ of the model parameters. The performance of the HMC is highly sensitive to the step size and number of steps in the leapfrog integrator for updating the position and momentum variables in a Hamiltonian dynamic simulation. NUTS was used, which is implemented in Stan and which extends HMC with adaptive tuning of both the step size and the number of steps in a leapfrog integration to sample efficiently from the posterior distributions. To overcome the inefficiency in the exploration of the posterior distribution of the model parameters, a reparameterization of the model parameters was used based on the map function from the model configuration space to the observed measurements. The reparameterization-based approach reduces the computation time by providing higher effective sample sizes and removing divergences by exploring the posterior distributions of the linear combinations of regional parameters that represent the eigenvectors obtained from the singular value decomposition of the gain matrix.

We denote the matrix of the eigenvectors of $G^T G$ as $V$ and the new parameters as $x_{i,0}^* = V^T x_{i,0}$ and $z_i(t_0)^* = V^T z_i(t_0)$.

The model identifiability was assessed based on an analysis of posterior samples, which demonstrates that the sampler explores all the modes in the parameter space efficiently. The analysis includes trace-plots (evolution of parameter estimates from draws over the iterations), pair plots (to identify collinearity between variables), and autocorrelation plots (to measure the degree of correlation between draws of samples). Sampling convergence of the algorithms is assessed by estimating the potential scale reduction factor and calculating the effective sample size based on the samples of the posterior model probabilities, providing estimates of the efficient run times of the algorithm.

Claims

1.  A method for estimating an epileptogenic zone network in a brain of an epileptic patient, comprising

    providing a virtual brain, the virtual brain being a computerized platform modelling various zones or nodes of a primate brain and connectivity between said zones or nodes;
    providing a mathematical model of an epileptogenic zone and of a propagation of an epileptic discharge from an epileptogenic zone to a propagation zone, the model describing the onset, the time-course and the offset of an epileptic discharge,
wherein the mathematical model is provided with epileptogenicity parameters;
loading the mathematical model in the virtual brain to create a virtual epileptic brain;
acquiring three-dimensional structural data representative of the anatomy of the brain of the epileptic patient;
personalising the virtual epileptic brain according to the structural data to obtain a personalised virtual epileptic brain model of the patient;
acquiring functional data recordings representative of epileptic seizures in the brain of the epileptic patient, the functional data recordings being acquired non-invasively by performing a deep brain temporal interference stimulation of the brain of the patient;
inverting the personalised virtual epileptic brain model of the patient to infer the epileptogenicity parameters specific to the patient for the various zones or nodes of the personalised virtual epileptic brain model of the patient;
simulating at least one epileptogenic seizure in the personalised virtual epileptic brain model wherein the epileptogenic parameters are inferred; and
estimating the epileptogenic zone network in the brain of the epileptic patient.

2. The method of claim 1, wherein the personalised virtual epileptic brain is a high-resolution model that simulates a neural source activity with a spatial resolution of less than 5 mm$^2$.

3. The method of claim 2, wherein the spatial resolution is of approximately 1 mm$^2$.

4. The method of one of the claims 1, 2 or 3, wherein, for performing the deep brain temporal interference stimulation of the brain of the patient, it is provided at least two pairs of stimulation electrodes (1, 2), each pair of electrodes providing an electric signal at a carrier frequency (f1, f2) generating an envelope frequency for stimulation at a point in space in the brain of the patient (C), the envelope frequency being equal to the difference $\Delta f$ between the two electric signal frequencies provided.

5. The method of one of the claims 1 to 4, wherein, for performing the deep brain temporal interference stimulation of the brain of the patient, it is provided at least four pairs of stimulation electrodes (1, 2, 3, 4), each pair of electrodes providing an electric signal at a carrier frequency (f1, f2, f3, f4), wherein the mean value between the carrier frequency (f1) of a first pair (1) of electrodes and the carrier frequency (f2) of a second pair (2) of electrodes defines a first mean carrier frequency, and the mean value between the carrier frequency (f3) of a third pair (3) of electrodes and the carrier frequency (f4) of a fourth pair (4) of electrodes defines a second mean carrier frequency, and wherein the difference between said first and second mean carrier frequencies is at least 200 Hz, preferably at least 500 Hz even more preferably at least 800 Hz.

6. The method of one of the claims 4 or 5 wherein each electric signal provided by each pair of electrodes (1, 2, 3, 4) has a carrier frequency (f1, f2, f3, f4) of at least 800 Hz, preferably at least 900 Hz, more preferably at least 1000 Hz.

7. The method of one of the claims 4, 5 or 6, wherein the electrodes are applied at different locations on a surface of a scalp of the patient skull.

8. The method of one of the claims 4 to 7, wherein the first pair of electrodes (1) and the second pair or electrodes (2) generate a first envelope frequency (A), said first envelope frequency being equal to the difference between the electric signal (f1) frequency provided by the first pair of electrodes (1) and the electric signal frequency (f2) provided by the second pair of electrodes (2) ;

the third pair of electrodes (3) and the fourth pair of electrodes (4) generate a second envelope frequency (B), said second envelope frequency being equal to the difference between the electric signal frequency (f1) provided by the third pair of electrodes (3) and the electric signal (f2) frequency provided by the fourth pair of electrodes (4); and wherein,
the first envelope frequency is equal to the second envelope frequency.

9. The method of one of the claims 1 to 8, wherein the structural data comprise geometry data of the cortex of the patient's brain and structural connectivity within the cortex of the patient's brain.

10. The method of claim 9, wherein the geometry data is extracted from T1-weighted MRI and the structural connectivity is extracted from tractography on diffusion weighted MRI data.

11. The method of one of the claims 1 to 8, further comprising

   providing functional data recordings acquired invasively, which are representative of epileptic seizures in the brain of the epileptic patient;
   providing an integration module;
   combining the functional data recordings acquired non-invasively by performing a deep brain temporal interference stimulation and the functional data recordings acquired invasively in the integration module.

12. The method of one of the claims 1 to 11, wherein the functional data recordings comprise electro-encephalographic data recordings acquired by performing temporal interference and electro-encephalographic data recordings acquired by stereo-electro-encephalography.

13. The method of one of the claims 1 to 12, wherein the mathematical model of an epileptogenic zone and of a propagation of an epileptic discharge from an epileptic zone to a propagation zone comprises a plurality of state variables and a parameter $I_{stim}$ representative of the stimulation time course.

14. The method of claim 13, wherein the mathematical model is provided by the set of equations as follows:

$$\dot{x}_1 \quad = \quad y_1 - f_1(x_1) - z + I_{ext1} + I_{stim}$$

$$\dot{y}_1 \quad = \quad c - dx_1^2 - y_1$$

$$\dot{z} \quad = \quad r(4(x_1 - x_0 + \mathrm{nH}(\mathrm{m} - m_{thresh})) - z) + f_3(z) + K \sum_{j=1}^{N} C_{i,j} \left( x_1^j - x_1^i \right)$$

$$\dot{m} \quad = \quad r(k|I_{stim}| - m)$$

where

$$f_1(x_1) \quad = \quad \begin{cases} ax_1^3 - bx_1^2 & \text{if } x_1 < 0 \\ -(m + 0.6(z-4)^2)x_1 & \text{if } x_1 \geq 0 \end{cases}$$

$$f_3(z) \quad = \quad \begin{cases} -0.1z^7 & \text{if } z < 0 \\ 0 & \text{if } z \geq 0 \end{cases}$$

*wherein the state variables $x_1$ and $y_1$ describe the activity of neural populations on a fast time scale and can model fast discharges, the oscillation of the slow permittivity variable z drives the system autonomously between ictal and interictal states, variable m describes the stimulation effect, the parameter $x_0$ indicates the degree of excitability and directly controls the dynamics of the neural population to produce the seizure or not, The default parameters are $I_{ext1}$ = 3.1, c = 1, d = 5, r = 0.00035, a = 1, b = 3, the coupling between nodes of the network is defined by $C_{i,j}$, which comes from the structural connectivity, K scales is the connectivity and, for stimulation effects, $I_{stim}$ describes stimulation input.*

15. The method according to one of the claims 1 to 14, wherein the brain model inversion implements Hamiltonian Monte Carlo sampling techniques from Bayesian inference methods.

16. A system for estimating an epileptogenic zone network in a brain of an epileptic patient, comprising

   a virtual brain, the virtual brain being a computerized platform modelling various zones or nodes of a primate brain and connectivity between said zones or nodes;
   a mathematical model of an epileptogenic zone and of a propagation of an epileptic discharge from an epileptic zone to a propagation zone, the model describing the onset, the time-course and the offset of an epileptic discharge, the mathematical model being provided with epileptogenicity parameters;

the mathematical model being loaded in the virtual brain to create a virtual epileptic brain;

acquired three-dimensional structural data representative of the anatomy of the brain of the epileptic patient;

the virtual epileptic brain being personalised according to the structural data to obtain a personalised virtual epileptic brain model of the patient;

a deep brain temporal interference stimulation device

acquired functional data recordings representative of epileptic seizures in the brain of the epileptic patient, the functional data recordings being acquired non-invasively by performing a deep brain temporal interference stimulation of the brain of the patient using the deep brain temporal interference stimulation device;

a module for inverting the personalised virtual epileptic brain model to infer the epileptogenicity parameters specific to the patient for the various zones or nodes of the personalised virtual epileptic brain model of the patient;

means for simulating at least one epileptogenic seizure in the personalised virtual epileptic brain model where the epileptogenic parameters are inferred; and

means for estimating the epileptogenic zone network in the brain of the epileptic patient.

A. TI : Adding two frequencies create an envelope

B. mTI : Adding two envelopes create a greater envelope

C. mTI : Reducing the overall amplitude increases focality

FIG. 1A

EP 4 449 976 A1

Fig. 1B

FIG. 1C

Fig. 1D

FIG. 2A

FIG. 2B

FIG. 3A

Low High

FIG. 3B

FIG. 3C

FIG. 3D

24

FIG. 3E

FIG. 3F

FIG. 3G

FIG. 4A

4310.0 ms   4540.0 ms   4560.0 ms

9380.0 ms   9980.0 ms   12980.0 ms

FIG. 4B

FIG. 4C

FIG. 4D

FIG. 4E

FIG. 4F

FIG. 5A

FIG. 5B

FIG. 5C

FIG. 5D

FIG. 6A

FIG. 6B

Application Number

EP 23 16 9009

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2021/197612 A1 (UNIV DAIX MARSEILLE AMU [FR]; INST NAT SANTE RECH MED [FR]) 7 October 2021 (2021-10-07) * page 2, line 32 – page 3, line 11 * * page 5, line 6 – page 6, line 31 * * page 12, lines 8-19 * * page 16, lines 26-28 * * page 19, line 4 – page 21, line 12 * * page 31, line 13 – page 33, line 22 * * claims; figures * ----- | 1-16 | INV. A61B5/00 |
| Y | SE 2 150 968 A1 (FRIGG AB [SE]) 24 January 2023 (2023-01-24) * paragraphs [0006], [0042] – [0058] * * claims; figures * ----- | 1-16 | |
| A | MAKHALOVA JULIA ET AL: "Virtual epileptic patient brain modeling: Relationships with seizure onset and surgical outcome", EPILEPSIA, [Online] vol. 63, no. 8, 1 August 2022 (2022-08-01) , pages 1942-1955, XP093082666, New York , US ISSN: 0013-9580, DOI: 10.1111/epi.17310 Retrieved from the Internet: URL:https://www.ncbi.nlm.nih.gov/pmc/artic les/PMC9543509/pdf/EPI-63-1942.pdf> * section 2.5 and 4.1 * ----- -/-- | 1-16 | TECHNICAL FIELDS SEARCHED (IPC) A61B G16H A61N |

The present search report has been drawn up for all claims

| Place of search | Date of completion of the search | Examiner |
|---|---|---|
| The Hague | 19 September 2023 | Bataille, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 23 16 9009

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CAO JIAMING ET AL: "STIMULUS: Noninvasive Dynamic Patterns of Neurostimulation Using Spatio-Temporal Interference", IEEE TRANSACTIONS ON BIOMEDICAL ENGINEERING, IEEE, USA, vol. 67, no. 3, 30 May 2019 (2019-05-30), pages 726-737, XP011772244, ISSN: 0018-9294, DOI: 10.1109/TBME.2019.2919912 [retrieved on 2020-02-19] * the whole document * ----- | 1-16 | |

TECHNICAL FIELDS
SEARCHED        (IPC)

The present search report has been drawn up for all claims

| Place of search | Date of completion of the search | Examiner |
|---|---|---|
| The Hague | 19 September 2023 | Bataille, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 23 16 9009

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2023

| Patent document cited in search report | | Publication date | Patent family member(s) | | Publication date |
|---|---|---|---|---|---|
| WO 2021197612 | A1 | 07-10-2021 | BR 112022019823 | A2 | 22-11-2022 |
| | | | CA 3172108 | A1 | 07-10-2021 |
| | | | CN 115668394 | A | 31-01-2023 |
| | | | EP 4128268 | A1 | 08-02-2023 |
| | | | IL 296981 | A | 01-12-2022 |
| | | | JP 2023528558 | A | 05-07-2023 |
| | | | KR 20230019248 | A | 07-02-2023 |
| | | | US 2023178250 | A1 | 08-06-2023 |
| | | | WO 2021197612 | A1 | 07-10-2021 |
| SE 2150968 | A1 | 24-01-2023 | SE 2150968 | A1 | 24-01-2023 |
| | | | WO 2023003501 | A1 | 26-01-2023 |

EPO FORM P0459

For more details about this annex : see Official Journal of the European Patent Office, No. 12/82

**EP 4 449 976 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JIRSA VK ; STACEY WC ; QUILICHINI PP ; IVANOV AI ; BERNARD C.** On the nature of seizure dynamics. *Brain,* 2014, vol. 137, 2210-2230